(19) Europäisches Patentamt
European Patent Office
Office européen des brevets

(11) Publication number: 0 468 638 A1

(12) # EUROPEAN PATENT APPLICATION

(21) Application number: 91305614.9

(51) Int. Cl.[5]: C07C 209/12

(22) Date of filing: 21.06.91

(30) Priority: 23.07.90 US 556686

(43) Date of publication of application:
29.01.92 Bulletin 92/05

(84) Designated Contracting States:
DE FR GB

(71) Applicant: TEXACO CHEMICAL COMPANY
3040 Post Oak Boulevard
Houston, Texas 77056(US)

(72) Inventor: Su, Wei-Yang
11814 Knights Bridge
Austin, Texas 78759(US)

(74) Representative: Brock, Peter William et al
UROUHART-DYKES & LORD 91 Wimpole Street
London W1M 8AH(GB)

(54) **A continuous process for preparing quaternary ammonium salts.**

(57) Long chain tertiary amines can be continuously converted into quaternary ammonium chlorides by passing the amines and alkyl chlorides over a metal oxide catalyst having Lewis acid sites.

Preferred amines are dihydrogenatedtallowalkylmethylamine, dimethylcocoalkylamine, dicocoalkylmethylamine, didodecyl methylamine or tridodecylamine.

Preferred catalysts comprise at least one oxide of a metal of Group IIA, IIIA, IIIB, IVA or IVB, such as oxide of silica, zirconia or titania, hafnium dioxide, alumina or a combination thereof.

EP 0 468 638 A1

This invention relates to an improved process for preparing quaternary ammonium salts, and more particularly to a continuous process for preparing quaternary ammonium chlorides by reacting a tertiary amine with an organic chloride in the presence of a heterogeneous catalyst.

The preparation of quaternary ammonium salts is known in the art. A good overview of the production of cationic surfactants from fatty nitriles is found in an article titled "Industrial Production of Fatty Amines and Their Derivatives", JAOCS, 61 No. 2 (Feb. 1984). It is noted that the largest quantity of salts produced, and the most important class of quaternary ammonium salts, is constituted by difattydimethyl ammonium salts, produced from secondary amines and methyl chloride, under continuous addition of alkali.

In addition, known methods of preparation of quaternary ammonium compounds are discussed at p.526 in Encyl. Chem. Techn. 3rd Ed. (Hoechst) 19:521(1982). Generally a suitable tertiary amine is reacted with an alkylating agent, such as an alkyl ester. There are many variations in the final product, because of the large number of diverse starting amines and alkylating agents. The tertiary amines used commercially are derived from synthetic or natural raw materials.

US-A-3175008 discloses a method for preparing tetraaliphatic ammonium chloride by mixing an aliphatic secondary amine, sodium bicarbonate and alkyl alcohol having 1 to 4 carbon atoms with methyl chloride at a temperature of at least 60-70 °C.

US-A-3377382 discloses novel fatty quaternary ammonium compounds, containing residual fatty acid monomers remaining after the polymerisation of higher unsaturated fatty acids, having substantially similar properties to dimethyl dihydrogenated tallow alkyl quaternaries, which are commonly used for their softening properties.

In the processes discussed above, the methods of production are not continuous. A discontinuous reaction is less efficient, because much more time is required to fill, heat and vent the reaction vessel. Also more of the alkyl chloride is lost as a result of repeated venting of the reaction vessel.

A continuous process for the manufacture of quaternary ammonium chlorides containing at least one aliphatic group of 8 to 22 carbons is disclosed in US-A-3813441. Amines are reacted with methyl chloride in the presence of an alkali metal hydroxide. That reaction is carried out in the presence of corresponding quaternary ammonium compounds, which decreased saponification of methyl chloride. Further, the use of alkali metal hydroxides in place of sodium bicarbonate considerably improved the colour of the quaternary ammonium compounds. The disadvantages of that process include corrosion caused by using caustic alkali, and also the need to dispose of a large amount of by-products.

It would be a distinct advance in the art if quaternary ammonium salts were continuously produced over a heterogeneous catalyst. If such a process offered high turnover rate, and high selectivity, such a process would fulfil a need in the art.

The present invention is concerned with the problem of providing a method of preparing quaternary ammonium salts by continuously reacting tertiary amines with alkyl chlorides in the presence of a catalyst.

In one embodiment, this invention provides a continuous process for preparing quaternary ammonium salts by reacting long chain tertiary amines with an alkyl chloride in the presence of a solvent, over a heterogeneous catalyst comprising a metal oxide with Lewis acid sites.
Preferably, the tertiary amine has the formula

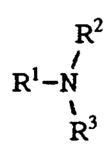

where $R^1$ is an aliphatic group having 8 to 22 carbon atoms and $R^2$ and $R^3$ are each an aliphatic group having 1 to 22 carbon atoms, and more preferably, the tertiary amine is dihydrogenatedtallowalkylmethylamine, dimethylcocoalkylamine, dicocoalkylmethylamine, didodecyl methylamine or tridodecylamine.

Preferably, the alkyl chloride is methyl chloride, benzyl chloride or ethyl chloride.

In a preferred embodiment, the heterogeneous catalyst comprises at least one oxide of an element of Group IIA, IIIA, IIIB, IVA or IVB of the Periodic Table. The form of the Periodic Table is that set out at page 789 of The Condensed Chemical Dictionary, 10th Edition (1981), (Van Nostrand Reinhold Company).

Preferably the catalyst is an oxide of silica, zirconia or titania, hafnium dioxide, alumina or a combination thereof.

In a preferred embodiment, the solvent is a hindered alcohol, most preferably isopropanol. Optionally, water can be used as a co-solvent.

According to the present invention, quaternary ammonium salts are produced in a continuous process

using metal oxides with Lewis acid sites as catalysts. Quaternary ammonium salts were obtained by reacting tertiary amines which had at least one long aliphatic chain with organic chlorides according to the following equation:

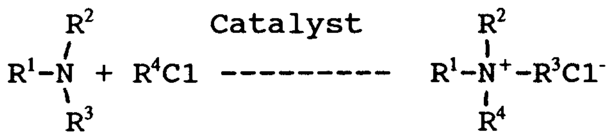


in which, for instance, in a preferred embodiment, $R^4$ is methyl or benzyl, $R^1$ represents an aliphatic radical having 8 to 22 carbon atoms, $R^2$ and $R^3$ represent an aliphatic radical of 1 to 22 carbon atoms.

An intermediate species during the reaction may be represented by

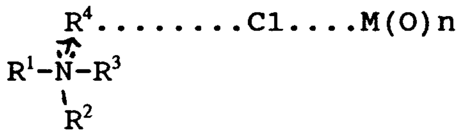

wherein M(O)n represents the catalyst.

The tertiary amines useful in this invention can be derived from synthetic or natural raw materials. The tertiary amines can generally be represented by the formula:

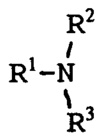

where $R^1$, $R^2$ and $R^3$ have the values defined above. Suitable tertiary amines include the asymmetrical methyl difatty amines $R_2N(CH_3)$ and dimethyl fatty amines of the formula $RN(CH_3)_2$, and those derived from primary or secondary amines by reaction with ethylene oxide.

The amines should contain at least one aliphatic group having 8 to 22 carbon atoms e.g. N,N-dimethyloctylamine, N,N-dimethyldecylamine, N,N-dimethylstearylamine, N,N-dimethyloleylamine, methyldioctylamine, methyldidecylamine and methyldistearylamine, and especially amines having different lengths of chains of a statistical proportion as obtained by hydrogenation of fatty nitriles produced from fatty acids.

Particularly useful amines include dihydrogenated tallowalkylmethylamine, dimethylcocoalkylamine, dicocoalkylmethylamine, didodecylmethylamine and tridodecylamine.

Suitable organic chloride alkylating agents include methyl chloride, ethyl chloride, and benzyl chloride.

It has been surprisingly discovered according to the present invention that metal oxides with Lewis acid sites provide excellent catalysts for the continuous reaction to produce quaternary ammonium salts.

Compounds which may be employed as catalysts include, but are not limited to, those containing elements of Group IIA, IIIA, IIIB, IVA and IVB of the Periodic Table. Suitable compounds include the oxides of aluminium, silicon, titanium, hafnium and zirconium or combinations thereof. Examples include alumina, silica(silicon dioxide), titania (titanium dioxide), hafnium dioxide and zironia, as well as combinations thereof. Good results were observed using, for example, Norton catalyst carrier SZ5445, containing mainly silica, zirconia and hafnium dioxide. The catalyst may be in the form of powders, pellets, spheres and extrudates.

Commercially available catalyst which fall within this definition include Norton SZ5445 and Norton 64775. Norton SZ5445 is a 5-8 mesh (2.38 to 4mm) catalyst incorporating $ZrO_2$-$SiO_2$-$HfO_2$. Norton 64775 is a titania catalyst carrier from Norton.

As solvents to be used for the quaternary ammonium compounds, and for further diluting the reaction medium, alcohols containing from 1 to 4 carbon atoms, preferably hindered alcohols, such as isopropanol, are suitable.

When a solvent is employed, an amount from 5 to 50% of the total content of the system is useful. An amount from 20 to 30% is preferred.

The reaction preferably takes place in a stainless steel tubular upflow reactor. Atmospheric pressure is

sufficient for the reaction to proceed. The reaction can occur at pressures of 0.1 to 21 MPa. Preferred pressures are in the range of 0.8 to 10.5 MPa. The temperature in the reactor should be 50 to 150 °C and preferably 70 to 130 °C.

The mole ratio of amine to chloride can be from 1:1.2 to 1.2:1, a ratio of 1:1 being preferred.

The examples demonstrate not only the benefits of a continuous system, but the improved conversion of amines observed using the process according to the invention. Example 1 demonstrates conversions of 99% or more which are obtained using the metal oxide catalyst in the continuous system.

## EXAMPLES

### EXAMPLE 1

**Preparation of Dimethyl Dihydrogenatedtallowalkyl Ammonium Chloride**

To a 550cc DOWTHERM$^R$ heated, stainless steel tubular upward flow reactor which had an internal diameter of 40mm(1.338") and a thermowell fabricated from 6.35 mm (1/4-inch) external diameter tubing extended upward into the catalyst bed was charged 550 cc of 2.38 to 4 mm (5-8 mesh) Norton SZ5445 catalyst carrier ($ZrO_2$-$SiO_2$-$HfO_2$ support).
Dihydrogenatedtallowalkylmethylamine under the tradename KEMAMINE$^R$ T-9701, sold by Witco Chemical, and methyl chloride/isopropanol solution were then run through the reactor bed at a total WHSV of 0.75 g/hr-cc and the reactant amine/chloride mole ratio of 1/1 with a 25% total isopropanol content. At 120 °C and 7 MPa (1000 psig), an off-white product was obtained. NMR spectrum analysis showed that 99 to 100% of the amines were converted into the corresponding ammonium chloride.

### EXAMPLE 2

**Titania**

The procedure of Example 1 was followed, except that Norton titania carrier (Norton 64775) was used. An off-white product was obtained, and NMR spectrum analysis showed that about 97% amine conversion was obtained.

### EXAMPLE 3

**Alumina**

The procedure of Example 1 was followed, except that alumina was used. A yellow product was obtained, and NMR spectrum analysis showed that about 95% amine conversion was obtained.

### EXAMPLE 4 (Comparison)

**Pyrex Glass Beads**

The procedure of Example 1 was followed, except that Pyrex glass beads were used. An off-white product was obtained, and NMR spectrum analysis showed that about 81% amine conversion was obtained. This is a comparative example which demonstrates that a catalyst with weaker Lewis acid sites has less catalytic effect.

### EXAMPLE 5

The procedure of Example 1 was followed, except the total WHSV was 1.10 g/hr-cc. An off-white product was obtained, and NMR spectrum analysis showed that about 89% amine conversion was obtained.

### EXAMPLE 6

**Preparation of Dimethylcocoalkylbenzylammonium Chloride**

To a 550cc DOWTHERM$^R$ heated, stainless steel tubular, upward flow reactor was charged 550cc of

alumina catalyst carrier. Dimethylcocoalkylamine, which was obtained from Ethyl Corp. under the tradename ADMA$^{R}$ 1214, and benzyl chloride/isopropanol solution were then run through the reactor bed, at a total WHSV of 0.87g/hr-cc and a reactant amine/chloride mole ratio of about 1:1, with a 25% total isopropanol content. At 120 °C and 7 MPa (1000 psig), a yellow product was obtained. NMR spectrum analysis showed that substantially all the amines were converted into the corresponding ammonium chloride.

## Claims

1. A continuous process for preparing quaternary ammonium salts characterized by reacting long chain tertiary amines with an alkyl chloride in the presence of a solvent, over a heterogeneous catalyst comprising a metal oxide with Lewis acid sites.

2. A process according to Claim 1 characterized in that the tertiary amine has the formula

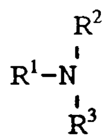

where $R^1$ is an aliphatic group having 8 to 22 carbon atoms and $R^2$ and $R^3$ are each an aliphatic group having 1 to 22 carbon atoms.

3. A process according to Claim 2 characterized in that the tertiary amine is dihydrogenatedtallowalkylmethylamine, dimethylcocoalkylamine, dicocoalkylmethylamine, didodecyl methylamine or tridodecylamine.

4. A process according to any one of claims 1 to 3 characterized in that the alkyl chloride is methyl chloride, benzyl chloride or ethyl chloride.

5. A process according to any one of claims 1 to 4 characterized in that the heterogeneous catalyst comprises at least one oxide of an element of Group IIA, IIIA, IIIB, IVA or IVB of the Periodic Table.

6. A process according to Claim 5 characterized in that the catalyst is an oxide of silica, zirconia or titania, hafnium dioxide, alumina or a combination thereof.

7. A process according to any one of Claims 1 to 6 characterized in that the solvent is a hindered alcohol.

8. A process Claim 7 characterized in that the solvent is isopropanol.

9. A process according to Claim 7 or 8 characterized in that water is used as a co-solvent.

10. A process according to any one of claims 1 to 9 characterized in that the mole ratio of amine to chloride is 1:1.2 to 1.2:1.

European Patent Office

# EUROPEAN SEARCH REPORT

Application Number

**EP 91 30 5614**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-3 175 008 (S.H. SHAPIRO)<br>- - - - - | 1 | C 07 C 209/12 |

| TECHNICAL FIELDS SEARCHED (Int. Cl.5) |
|---|
| C 07 C |

The present search report has been drawn up for all claims

| Place of search | Date of completion of search | Examiner |
|---|---|---|
| The Hague | 04 November 91 | PAUWELS G.R.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document